Europäisches Patentamt

European Patent Office

Office européen des brevets

(11) Veröffentlichungsnummer: **0 498 924 A1**

# EUROPÄISCHE PATENTANMELDUNG

(21) Anmeldenummer: **91115070.4**

(22) Anmeldetag: **09.09.91**

(51) Int. Cl.5: **A61K 7/06**

Die Anmeldung wird, wie ursprünglich eingereicht, unvollständig veröffentlicht (Art. 93 (2) EPÜ). Die Stelle der Patentansprüche, die offensichtlich eine Auslassung enthält, ist als Lücke an der entsprechenden Stelle ersichtlich.

(30) Priorität: **10.09.90 DE 4028696**

(43) Veröffentlichungstag der Anmeldung:
**19.08.92 Patentblatt 92/34**

(84) Benannte Vertragsstaaten:
**AT BE CH DE DK ES FR GB GR IT LI LU NL SE**

(71) Anmelder: **Ahmad, Khalil**
**Im Füldchen 20**
**W-6000 Frankfurt 90(DE)**

(72) Erfinder: **Ahmad, Khalil**
**Im Füldchen 20**
**W-6000 Frankfurt 90(DE)**

(74) Vertreter: **Höly, Albert et al**
**Grosse Bockenheimer Strasse 41**
**W-6000 Frankfurt am Main 1(DE)**

(54) **Haarwuchsmittel in Ölform und Haarwasser.**

(57) Ein Haarwuchsmittel mit Pflanzenextrakten verbesserter Wirksamkeit enthält eine Kombination folgender Grundsubstanzen:
1 - 30 ml Wacholderöl auf pflanzlicher Basis
2 - 30 ml Baldrianöl
6 - 10 ml Birkenhaaröl auf Paraffinbasis
6 - 10 ml Johanniskrautöl auf pflanzlicher Basis
6 - 10 ml Klettenwurzelhaaröl auf Paraffinbasis
6 - 12 ml Rosenhaaröl auf Paraffinbasis
2 - 5 ml Arnikaöl, welches zu 90 % aus Olivenöl besteht, auf pflanzlicher Basis
0,1 - 1,2 g Pfefferminzblätter
0,2 - 3 Stück süße Mandeln ohne Schale
0,1 - 1,2 g Brennesselblätter
15 - 70 Stück Radies-Eiszapfen-Samen
20 - 80 Stück Kohlrabisamen
15 - 70 Stück Bockshornkleesamen
15 - 70 Stück gelbe Senfkörner
2 - 8 Stück à 1 cm$^3$ Apfel.

EP 0 498 924 A1

Die Erfindung betrifft ein Haarwuchsmittel in Ölform nach dem Oberbegriff des Anspruchs 1 sowie ein Haarwasser nach dem Oberbegriff des Anspruchs 8.

Es ist bekannt, zur Steigerung der Kopfhautdurchblutung und Förderung des Haarwuchses insbesondere Haarwässern Pflanzenextrakte zuzusetzen. Solche Pflanzenextrakte sind Blätter-, Wurzel- und Blütenauszüge von Birke, Brennessel, Arnika, Kletten, Hamamelis. Die Wirksamkeit solcher Pflanzenextrakte in bisher üblichen Zusammensetzungen ist jedoch fraglich.

Bekannt ist es auch, daß hohe Dosen von Corticosteroiden oder eine Chlordinitrobenzolanwendung wieder einen Haarwuchs bewirken kann, jedoch nur für die Dauer der Behandlung. Bei den letztgenannten Substanzen befürchtet ein Teil der Anwender Nebenwirkungen, so daß sie insofern Mittel auf pflanzlicher Grundlage nicht ersetzen können (Römpps Chemielexikon, Stuttgart 1983, Seite 1580 - 1584).

Der vorliegenden Erfindung liegt daher das seit langem erkannte Bedürfnis zugrunde, ein Haarwuchsmittel mit Pflanzenextrakten verbesserter Wirksamkeit zu schaffen.

Diese Aufgabe wird durch die in dem kennzeichnenden Teil des Anspruchs 1 angegebene Zusammensetzung der Grundsubstanzen bzw. Wirkstoffe gelöst, die teilweise in Ölform vorliegen.

Dieses Haarwuchsmittel ist sehr wirksam und läßt Haare auch an den Kopfstellen nachwachsen, an denen die behandelnde Person ihre Haare schon lange verloren hat.

Das Haarwuchsmittel ist weitgehend auf pflanzlicher Grundlage zusammengesetzt. Allerdings können einige der Grundsubstanzen Pflanzenextrakte darstellen, die mit anderen Trägerstoffen vermengt sind, die insbesondere auf Paraffinbasis hergestellt sind. Dies gilt für Baldrianöl, welches entweder zu 100 % auf pflanzlicher Basis hergestellt ist oder auf Paraffinbasis oder je hälftig auf pflanzlicher Basis und Paraffinbasis. - Das gleiche gilt für Apfelöl, welches gemäß Anspruch 2 in der Menge 0,25 - 10 ml statt der Apfelstücke in dem Haarwuchsmittel eingesetzt werden kann. - Erwähnenswert ist zu der Zusammensetzung nach Anspruch 1 das Arnikaöl, welches zum Beispiel aus 1 ml reinem Arnikaöl und 9 ml Olivenöl zusammengemischt ist. Das Birkenhaaröl, das Klettenwurzelhaaröl, das Rosenhaaröl beruhen jeweils auf Paraffinbasis. Die Pfefferminzblätter und die Brennesselblätter können jeweils im getrockneten Zustand oder frisch zugesetzt werden. Eine besonders bevorzugte Bemessung der Bestandteile des Haarwuchsmittels ist im einzelnen in Anspruch 3 angegeben. Diese Zusammensetzung kann jedoch bei noch guter Wirksamkeit variabel erfolgen, wobei jede der angegebenen Grundsubstanzen in einem Bereich von ± 0 - 30 % von den angegebenen speziellen Werten abweichen darf.

Auch ist bei der Zusammensetzung gemäß Anspruch 3 ein Ersatz der Apfelstücke durch Apfelöl, wie in Anspruch 4 angegeben, möglich.

Die Wirksamkeit des Haarwuchsmittels nach Anspruch 1 kann noch durch die Zusatzkomponenten gemäß Anspruch 5 gesteigert werden. Da insbesondere Kokosnußöl jedoch nicht für jeden Benutzer verträglich ist, werden diese Zusatzkomponenten getrennt konfektioniert, um von dem Benutzer vor Gebrauch den Grundsubstanzen nach Anspruch 1 beigegeben zu werden. Bei negativer Wirkung können jedoch die Zusatzkomponenten weggelassen werden. - Das gleich gilt für die Zusatzkomponenten, die gemäß Anspruch 6 in anderer Bemessung der Mischung der Grundsubstanzen gemäß Anspruch 3 beigefügt werden können.

Ein Haarwasser, welches weiteren Haarsausfall verhindert und den Neuwuchs fördert, enthält im wesentlichen die gleichen Wirkstoffe wie das beschriebene erfindungsgemäße Haarwuchsmittel. Demgemäß sind für das Haarwasser in Anspruch 8 die Grundsubstanzen angegeben, aus denen die Wirkstoffe hergestellt werden können. Hierbei wird unter variabler Zusammensetzung verstanden, daß jede einzelne Grundsubstanz in dem Bereich von 0 - ± 50 % mengenmäßig geändert werden darf, mit Ausnahme der genannten Rosenblüten, der Klettenwurzel und der Wacholderbeeren. Für die letztgenannten drei Grundsubstanzen besteht eine Änderungsmöglichkeit von 0 - +20 % und -80 %. Die Rosenblüten können mit oder ohne Blütenansatz verwendet werden.

In Anspruch 9 sind Zusatzsubstanzen für das Haarwasser genannt, die der Steigerung der Wirksamkeit dienen, aber auch einzeln oder in beliebiger Kombination weggelassen werden können.

Sämtliche Angaben zum Gehalt bzw. deren Änderung nach Anspruch 8 gehen von einem gleichmäßigen Anteil aller Grundsubstanzen aus. Der Anteil der Zusatzsubstanzen nach Anspruch 9 muß jedoch geringer sein, und zwar 0 - 20 % eines auf alle Substanzen bezogenen Durchschnittswerts, der im folgenden erläutert wird: Wenn beispielsweise das Haarwasser insgesamt 16 Substanzen (Grundsubstanzen und Zusatzsubstanzen) enthält, ist der Durchschnittswert zu berechnen aus dem gewünschten Gesamtgewicht G der Substanzen geteilt durch deren Anzahl, hier 16. Daraus ergibt sich der Durchschnittswert G/16 und für die Zusatzsubstanzen nach Anspruch 9: 0 - 20 % von G/16.

Nach Anspruch 10 können 1 - 10 g eines dieserart zusammengesetzen Auszugs der Grundsubstanzen und Zusatzsubstanzen in 100 - 250 ml 20 %igem bis 80 %igem Alkohol zugemischt werden.

Zu einzelnen Grundsubstanzen ist noch zu spezifizieren, daß der Kohlrabisamen nach Anspruch 11 besonders wirkungsvoll derjenige der blauen Delikateß-Kohlrabisorte ist.

Als Apfelsorte, aus der die Apfelstücke oder das Apfelöl bestehen, wird bevorzugt die Sorte Red Delicious verwendet.

Aus den Unteransprüchen 13 - 15 ergeben sich (weitere) vorteilhafte Merkmale.

Zur Herstellung des Haarwassers werden die Grundsubstanzen in den angegebenen Mischungsverhältnissen gemischt, und aus dieser Mischung wird ein Auszug hergestellt.

Diesem Auszug wird Alkohol in dem in Anspruch 10 angegebenen Mengenverhältnis zugemischt.

Fünf der zu dem Haarwuchsmittel gemäß Anspruch 1 und gemäß Anspruch 3 verwendeten Substanzen werden in folgender Weise hergestellt:

1. Rosenhaaröl:

Es werden die Wirkstoffe aus 0,2 - 3 g Rosenblüten (ohne Blütenansatz) in 50 ml Paraffin Perliquidum (dünnflüssig) gemischt.

2. Birkenhaaröl:

Es werden die Wirkstoffe aus 0,2 - 3 g Birkenblätter in 50 ml Paraffin Perliquidum (dünnflüssig) gemischt.

3. Klettenwurzelhaaröl:

Es werden die Wirkstoffe aus 1 - 5 g Klettenwurzel in 50 ml Paraffin Perliquidum (dünnflüssig) gemischt.

4. Baldrianöl:

Es werden die Wirkstoffe aus 0,5 - 5 g Baldrianwurzel in 50 ml Paraffin Perliquidum (dünnflüssig) gemischt.

5. Apfelöl:

Es werden die Wirkstoffe aus 2 - 10 g Apfel (ohne Schale) in 50 ml Paraffin Perliquidum (dünnflüssig) gemischt.

Die in dem Haarwuchsmittel nach Anspruch 1 oder Anspruch 3 einzusetzenden Samen können in Wasser eingeweicht werden, bevor sie den Mischungen der Öle zugesetzt werden.

Statt der bevorzugten Apfelsorte Red Delicious können andere Apfelsorten verwendet werden.

Die Behandlung erfolgt mit dem Haarwuchsmittel nach Anspruch 2. Das Mischungsverhältnis kann in dem Bereich von ± 0 bis 40 % von dem Verhältnis 1:1 abweichen. Zwei Tage darauf beginnt ein neuer Zyklus wie oben angeben. Ein- bis zweimal innerhalb von 14 Tagen soll auf die Kopfhaut nur Rosenhaaröl aufgetragen werden. Die Einwirkungsdauer des Haarwuchsmittels soll jeweils wenigstens 4 - 5 Stunden, vorzugsweise über Nacht, erfolgen. - Vor jeder Behandlung soll der Kopf etwa 40 Sek. shamponiert werden. Dann wird das Haarwuchsmittel aufgetragen. Das Haarwuchsmittel wirkt die ganze Nacht ein. Am nächsten Morgen wird das Haar mit normalem Shampoo gewaschen und das Haarwasser aufgetragen.

**Patentansprüche**

**1.** Haarwuchsmittel in Ölform nach Patent (Patentanmeldung P 41 21 778.0-41) mit Grundsubstanzen in folgender Kombination:
1 - 30 ml Wacholderöl auf pflanzlicher Basis
2 - 30 ml Baldrianöl
6 - 10 ml Birkenhaaröl auf Paraffinbasis
6 - 10 ml Johanniskrautöl auf pflanzlicher Basis
6 - 10 ml Klettenwurzelhaaröl auf Paraffinbasis
6 - 12 ml Rosenhaaröl auf Paraffinbasis
2 - 5 ml Arnikaöl, welches zu 90 % aus Olivenöl besteht, auf pflanzlicher Basis
0,1 - 1,2 g Pfefferminzblätter
0,2 - 3 Stück süße Mandeln ohne Schale
0,1 - 1,2 g Brennesselblätter
15 - 70 Stück Radies-Eiszapfen-Samen
20 - 80 Stück Kohlrabisamen
15 - 70 Stück Bockshornkleesamen
15 - 70 Stück gelbe Senfkörner
2 - 8 Stück à 1 cm$^3$ Apfel, (oder Apfelöl 2-10 ml)
**dadurch gekennzeichnet,**
daß zusätzlich folgende Substanzen enthalten sind:
15 - 30 ml Traubenkernöl auf pflanzlicher Basis
10 - 28 ml Orangenblütenöl auf pflanzlicher Basis
1 - 12 ml Honigmelonenöl auf Paraffinbasis.
2 - 56 ml Kokosnußöl auf pflanzlicher Basis
1 - 40 ml Linsen-Uridöl
1 - 40 ml Kiwiöl auf Paraffinbasis oder pflanzlicher Basis
1 - 40 ml Korianderöl auf Paraffinbasis oder pflanzlicher Basis
1 - 40 ml Pfirsichöl auf Paraffinbasis oder pflanzlicher Basis

**2.** Haarwuchsmittel in Ölform nach Patent (Patentanmeldung P 41 21 778.0-4) mit folgenden Bestandteilen in variabler Zusammensetzung: Änderungsmöglichkeit + 0 - 30 %
28 ml Wacholderöl auf pflanzlicher Basis
28 ml Baldrianöl
6 ml Birkenhaaröl auf Paraffinbasis
6 ml Johanniskrautöl auf pflanzlicher Basis
6 ml Klettenwurzelhaaröl auf Paraffinbasis
6 ml Rosenhaaröl auf Paraffinbasis
2 - 3 ml Arnikaöl in einem Gemisch, welches zu 90 % aus Olivenöl besteht, auf pflanzlicher Basis
0,1 - 0,25 g Pfefferminzblätter
0,3 - 1 Stück süße Mandeln ohne Schale
0,1 -0,25 g Brennesselblätter
25 Stück Radies-Eiszapfen-Samen
35 Stück Kohlrabikleesamen
25 Stück Bockshornkleesamen
30 Stück gelbe Senfkörner
2 - 6 Stück á 1 cm$^3$ Apfel ohne Schale der Sorte Red Delicious, (oder Apfelöl 2-10 ml)

**dadurch gekennzeichnet,**

daß zusätzlich folgende Suhstanzen enthalten sind:

28 ml Kokosnußöl

28 ml Traubenkernöl auf pflanzlicher Basis

28 ml Orangenblütenöl auf pflanzlicher Basis

1 - 10 ml Honigmelonenöl auf Paraffinbasis

1 - 40 ml Linsen-Uridöl auf Paraffinbasis

1 - 40 ml Kiwiöl auf Paraffinbasis oder pflanzlicher Basis

1 - 40 ml Korianderöl auf Paraffinbasis oder pflanzlicher Basis

1 - 40 ml Pfirsichöl auf Paraffinbasis oder pflanzlicher Basis

**3.** Haarwuchsmittel nach Anspruch 1 oder 2,

**dadurch gekennzeichnet,**

daß alle Bestandteile in einem einzigen Behältnis aufbewahrt sind.

**4.** Haarwuchsmittel nach einem der vorangehenden Ansprüche,

**dadurch gekennzeichnet,**

daß Honigmelonenöl aus einem Wirkstoff, der aus 4 - 10 Stücken von je 1 cm$^3$ der geschälten Honigmelone oder aus 1 - 4 g Kernen des Samens der Honigmelone hergestellt ist, gemischt in 40 ml dünnflüssigem Paraffin, besteht.

**5.** Haarwuchsmittel nach Anspruch 1 oder 2,

**dadurch gekennzeichnet,**

daß das Pfirsichöl aus dem Wirkstoff auf 6 - 12 g Pfirsich in 50 ml dünnflüssigem Paraffin gemischt besteht.

**6.** Haarwasser nach Patent (Patentanmeldung P 41 21 778.9-14) mit folgenden Grundsubstanzen in variabler Zusammensetzung:

Birkenblätter

Johanniskraut

Pfefferminzblätter

Brennesselblätter

Bockshornkleesamen

Radies-Eiszapfen-Samen

Gelbe Senfkörner

Kohlrabisamen

Apfel ohne Schale

Rosenblüten

Klettenwurzel

Wacholderbeeren,

gegebenenfalls mit folgenden Zusatzsubstanzen:

Arnikablüten

Oliven

Baldrianwurzel

Süße Mandeln ohne Schale,

**dadurch gekennzeichnet,**

daß zusätzlich beigegeben sind:

Traubenkerne

Orangenblüten oder Orangenschalen

geschälte Honigmelone oder Honigmelonensamenkerne

Kokosnuß

Pfirsich oder Pfirsichblüten

Kiwi-Frucht und Kiwi-Samen

Koriander.

**7.** Haarwasser nach Anspruch 6,

**dadurch gekennzeichnet,**

daß zusätzlich Linsen-Urid enthalten ist.

**8.** Haarwasser nach den Ansprüchen 6 und 7,

**gekennzeichnet durch**

folgende Zusammensetzung:

a) Änderungsmöglichkeit innerhalb von 0 bis ± 50 % eines fiktiven gleichmäßigen Anteils aller Substanzen:

Birkenblätter

Johannisbeerblätter

Pfefferminzblätter

Brennesselblätter

Bockshornkleesamen

Radies-Eiszapfen-Samen

Gelbe Senfkörner

Kohlrabisamen

Apfel ohne Schale

Koriander

b) Änderungsmöglichkeit innerhalb von + 20 bis - 80 % eines fiktiven gleichmäßigen Anteils aller Substanzen:

Rosenblüten

Klettenwurzeln

Wacholderbeeren

Traubenkerne

Orangenblüten oder Orangenschalen

geschälte Honigmelone oder Honigmelonenkerne

Kokosnuß

Pfirsich oder Pfirsichblüten

Kiwi-Frucht oder Kiwi-Samen

c) Gehalt von 0 - 40 % eines auf alle Substanzen bezogenen fiktiven Durchschnittswertes:

Arnikablüten

Oliven

Baldrianwurzel

Süße Mandeln ohne Schale

Linsen-Urid.

**9.** Haarwasser nach Anspruch 8,

**dadurch gekennzeichnet,**

daß ein Auszug aus 1 bis 10 g Mischung der Substanzen (Grundsubstanzen und Zusatzsubstanzen) in 100 bis 250 ml 20 % bis 80%igem Alkohol gemischt sind und aus Apfel gewonnener Saft mit 100 - 250 ml 20 - 80%igem Alkohol gemischt wird und dem Haarwasser zugesetzt wird und daß aus bis zu 40 g Apfel gewonnener Saft der angegebenen Alkoholmenge zugesetzt ist.

**12.** Haarwasser nach einem der Ansprüche 8 - 11,

**dadurch gekennzeichnet,**

daß anstelle des geschälten Apfels Apfelblüten der Sorte Red Delicious als Grundsubstanz verwendet werden.

**13.** Haarwasser nach Anspruch 8,

**dadurch gekennzeichnet,**

daß es einen Auszug aus gleichen gewichtsmäßigen Anteilen der Grundsubstanzen enthält mit Ausnahme von Rosenblüten, Klettenwurzel und Wacholderbeeren, deren Anteil niedriger sein kann.

**14.** Haarwasser nach Anspruch 13,
**dadurch gekennezeichnet,**
daß der gewichtsmäßige Anteil einer der Grundsubstanzen in dem Auszug mit Ausnahme von Rosenblüten, Klettenwurzel und Wacholderbeeren um ± 50 % ausgehend von einem gleichmäßigen Anteil der Grundsubstanz in dem Auszug variiert ist.

**15.** Haarwasser nach einem der Ansprüche 11 - 14,
**dadurch gekennzeichnet,**
daß aus 1 - 10 g Apfel der Sorte Red Delicious gewonnener Saft mit 100 - 250 ml 20 - 80 %igem Alkohol gemischt wird und dem Haarwasser zugesetzt wird.

Europäisches Patentamt

**EUROPÄISCHER RECHERCHENBERICHT**

Nummer der Anmeldung

## EINSCHLÄGIGE DOKUMENTE

EP 91115070.4

| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
|---|---|---|---|
| A | AT - B - 381 449 (M. GUTWEIN) * Gesamt * -- | 1,2,6, 8,9, 12-15 | A 61 K 7/06 |
| A | FR - A - 2 338 035 (E. GUTTENBERG) * Gesamt * -- | 1,2,6, 8,9 | |
| A | CH - A - 179 254 (R. KRUMMEN) * Gesamt * -- | 1,2,3, 6,8,9, 13,14 | |
| A | DE - A - 3 039 281 (HERBARIA ORSZAGOS GYOGYNÖVENYFORGALMI KÖZÖS VALLALAT) * Patentansprüche * -- | 1,2,3, 6,8,9 | |
| A | DAS GROSSE REZEPTBUCH DER HAUT- UND KÖRPERPFLEGEMITTEL, 2. Auflage, 1956, K. ROTHEMANN, Dr. Alfred Hüthig Verlag, Heidelberg Seiten 37-57,67-87,306-322 * Seiten 37-55,69-86, 312-322 * ---- | 1-9, 12-15 | |

RECHERCHIERTE SACHGEBIETE (Int. Cl.⁵)

A 61 K 7/00

Der vorliegende Recherchenbericht wurde für alle Patentansprüche erstellt.

| Recherchenort | Abschlußdatum der Recherche | Prüfer |
|---|---|---|
| WIEN | 19-12-1991 | IRMLER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82